# EUROPÄISCHE PATENTANMELDUNG

(11) **EP 2 465 496 A1**
(43) Veröffentlichungstag der Anmeldung: **20.06.2012**
(21) Anmeldenummer: 10192111.2
(22) Anmeldetag: 22.11.2010
(51) Int. Cl.: A61K 9/48, A61K 47/44, A61K 33/10

(54) **Anti-azidotische pharmazeutische Zusammensetzung**

(71) Anmelder: Salmon Pharma GmbH, 4001 Basel (CH)
(72) Erfinder: Ammer, Richard, 58644, Iserlohn (DE); Poestges, Reiner, 58730, Froendenberg (DE); Ballmann, Christina, 48167, Münster (DE)
(74) Vertreter: Isarpatent

(57) **Zusammenfassung**

Die vorliegende Erfindung betrifft eine pharmazeutische Zusammensetzung zur peroralen Verabreichung, die Erdalkalimetall- oder Alkalimetallcarbonate eingebettet in einer lipophilen Matrix aufweist und wenigstens zwei Überzugsschichten aufweist. Die vorliegende Erfindung betrifft weiterhin die Verwendung dieser pharmazeutischen Zusammensetzung zur Behandlung einer metabolischen Azidose oder einer Übersäuerung des Blutes bei chronischer Niereninsuffizienz. Die vorliegende Erfindung betrifft weiterhin ein Verfahren zur Herstellung einer solchen pharmazeutischen Zusammensetzug.

## Beschreibung

Die vorliegende Erfindung betrifft eine pharmazeutische Zusammensetzung zur peroralen Verabreichung, die Erdalkalimetall- oder Alkalimetallcarbonate eingebettet in einer lipophilen Matrix aufweist und wenigstens zwei Überzugsschichten aufweist. Die vorliegende Erfindung betrifft weiterhin die Verwendung dieser pharmazeutischen Zusammensetzung zur Behandlung einer metabolischen Azidose oder einer Übersäuerung des Blutes bei chronischer Niereninsuffizienz. Die vorliegende Erfindung betrifft zudem ein Verfahren zur Herstellung einer solchen pharmazeutischen Zusammensetzug.

### Hintergrund der Erfindung

Unter einer Azidose wird ein Zustand der Übersäuerung des menschlichen oder tierischen Körpers verstanden. Liegt der pH-Wert im Blut unterhalb von 7,36 spricht man von einer Azidose. Der normal-pH-Wert des Blutes liegt im Bereich von 7,36 bis 7,44. Bei darüberliegenden pH-Werten liegt eine Alkalose vor.

Unter einer metabolischen Azidose versteht man die Ansammlung einer zu großen Menge saurer Stoffwechselprodukte im Blut, wie beispielsweise bei Zuckererkrankung oder chronischer Nierenerkrankung. Der pH-Wert sinkt ab, wenn die Pufferkapazität des Blutpuffers gegenüber Säuren erschöpft ist. Dies führt zu einem plötzlichen, starken Absinken des pH-Wertes und zu einer akuten Übersäuerung. Es kann sich dabei oftmals um akut lebensbedrohliche Zustände handeln.

Die häufigsten Ursachen für eine metabolische Azidose sind Niereninsuffizienz oder ein isulierter tubulärer H⁺-Sekretionsdefekt der Nieren, eine diabetische Stoffwechselentgleisung, Urämie oder eine Vergiftung mit sauren Substanzen, wie beispielsweise Acetylsalicylsäure. Ein Patient mit metabolischer Azidose fällt vor allem durch seine verstärkte, tiefe und beschleunigte Atmung auf. Bei der Ketoazydose des Diabetikers ist unter Umständen ein charakteristischer Acetongeruch in der Atemluft zu bemerken. Entscheidend für die Erkennung und Quantifizierung der metabolischen Azidose ist eine Blutgasanalyse, wobei sich aus dem Basendefizit, dem pH-Wert und dem CO₂-Partialdruck leicht das Ausmaß der metabolischen Azidose und das Ausmaß der respiratorischen Kompensation des Körpers erkennen lässt.

Abgesehen von kausalen Therapien, wie beispielsweise der Gabe von Insulin bei der diabetischen Ketoazidose, besteht die Therapie bei der metabolischen Azidose bzw. der Übersäuerung des Blutes in der Gabe von basischen Substanzen, beispielsweise Natriumhydrogencarbonat oder anderen Puffersubstanzen, mit denen derartige Stoffwechselentgleisungen zumindest vorübergehend eingegrenzt werden können.

Entsprechende Medikamente sind bereits auf dem Arzneimittelmarkt erhältlich. Zum Beispiel vertreibt die Firma Medice, Iserlohn das Produkt Nephrotrans^{®}, das pro Kapsel einen Gehalt von 500 bzw. 840 mg Natriumhydrogencarbonat aufweist. Diese Kapseln sind mit Hilfe eines Phthalat-Lackes magensaftresistent überzogen und lösen sich erst im Dünndarm auf. Dies führt zu einer Anhebung des Plasmahydrogencarbonatspiegels und zur Behebung eines Hydrogencarbonatdefizites zur Pufferung einer zu hohen Wassterstoffionenkonzentration und zu einer Steigerung des Blut-pH bei metabolischer Azidose.

Eine Freisetzung von Natriumhydrogencarbonat im Magen muss unbedingt vermieden werden, da Hydrogencarbonat durch das in der Magensäure enthaltene HCl zu Natriumchlorid und Kohlendioxidgas umgewandelt wird und somit für jedwede therapeutische Anwendung (d.h. zur Anhebung des Blut-pH) nicht mehr zur Verfügung steht. Insbesondere können sich Probleme bei postprandialer Einnahme des Arzneimittels ergeben, da dann, abhängig von der zugeführten Nahrung, der pH des Magens bis auf Werte von sogar pH 6,0 ansteigen kann, woraus sich ein verfrühtes Auflösen der magensaftresistenten Beschichtung ergeben kann. Hierdurch kann sich bei Ulcus-Patienten durch das entstehende Kohlendioxidgas sogar die Gefahr einer Ruptur ergeben. Zudem kann es, ausgelöst durch übermäßige CO₂-Freisetzung im Magen, zu einem ausgeprägten Ructus kommen.

Das oben genannte Präparat Nephrotrans^{®} weist eine besondere Galenik auf, die Natriumhydrogencarbonat direkt an den physiologischen Resorptionsort im Dünndarm transportiert. Im Produkt Nephrotrans^{®} ist das Natriumhydrogencarbonat in einer Mischung fetthaltiger Materialien enthalten, wobei die fetthaltigen Materialien eine pastöse Form aufweisen. Diese pastöse Zubereitung ist in Weichgelatinekapseln enthalten, die ihrerseits mit einem Phthalat-Lack magensaftresistent überzogen sind. Nach Verabreichung wird nach Passage des Magens und Auflösung der magensaftresistenten Beschichtung ein langsames Herauslösen von Natriumhydrogencarbonat aus der lipophilen Matrix erfolgen.

In gängigen Darreichungsformen werden als Material für die magensaftresistente Überzugsschicht Phthalsäureester (Phthalate) eingesetzt. Beispielsweise enthalten gängige magensaftresistente Beschichtungen große Mengen an Dibutylphthalat und Hypromellosephthalat (Hydroxypropylmethylcellulosephthalat). Phthalate sind jedoch gesundheitlich problematische Verbindungen, da sie im Verdacht stehen, wie Hormone zu wirken und beispielsweise Unfruchtbarkeit, Übergewicht und Diabetes hervorzurufen. Insofern existieren Bestrebungen, Phthalate vollkommen aus den verschiedenen Verbraucherprodukten, insbesondere jedoch auch Medikamenten, zu entfernen und durch funktionsgleiche und gesundheitlich unbedenkliche Komponenten zu ersetzen.

Im Zusammenhang mit der Verabreichung von Natriumhydrogencarbonat zur Bekämpfung der Übersäuerung des Blutes hat sich hierbei das Problem ergeben, dass vergleichbare magensaftresistente Überzüge, wie beispielsweise diejenigen aus der Gruppe der Eudragite, teilweise zu funktionsunfähigen magensaftresistenten Überzügen führten: Der Versuch, phthalathaltige Überzugsmaterialien ausschließlich durch andere gängige funktionale Überzugsmaterialien zu ersetzen, erwies sich als qualitativ unzureichend. Zerfallsprüfungen nach Ph.Eur. hatten ergeben, dass diese neuen Darreichungsformen häufig bereits unmittelbar nach der Herstellung OOS- (out of specification) Ergebnisse aufwiesen. Im Verlauf der Lagerzeit nahmen diese weiter zu, auch eine Anhebung der Filmdicken der magensaftresistenten Überzüge zeigte kein Verbesserungspotential.

Diese Probleme sind vermutlich darauf zurückzuführen, dass die gängigen Darreichungsformen (z.B. Weich- oder Hartgelatinekapseln) aufgrund ihres Aufbaus Unebenheiten aufweisen (beispielsweise Nähte). Diese erweisen sich beim Überziehen als Schwachstellen und führen so zu nicht spezifikationskonformer Freisetzung.

Insofern haben sich alle bislang unternommenen Versuche, Phthalat-freie magensaftresistent überzogene pharmazeutische Zusammensetzungen zur Behandlung einer Übersäuerung des Blutes herzustellen als nicht zielführend erwiesen. Insbesondere in Hinblick auf die oben geschilderte, unbedingt notwendige Vermeidung der Freisetzung von Natriumhydrogencarbonat im Magen muss das Funktionieren des magensaftresistenten Überzugs auf der pharmazeutischen Darreichungsform mit Sicherheit gewährleistet sein.

### Zusammenfassung der Erfindung

Insofern liegt der vorliegenden Erfindung die Aufgabe zu Grunde, eine pharmazeutische Zusammensetzung zur Behandlung einer Übersäuerung des Blutes bereitzustellen, die einerseits einen (untoxischen) magensaftresistenten Überzug aufweist, und die andererseits den Arzeibuchspezifikationen entspricht und eine Freisetzung von Natriumhydrogencarbonat im Magen effektiv verhindert.

Diese Aufgaben werden durch den Gegenstand von Anspurch 1 gelöst. Bevorzugte Ausführungsformen sind in den abhängigen Ansprüchen angegeben.

Die vorliegende Erfindung erreicht die Lösung der gestellten Aufgabe dadurch, dass wenigstens zwei Überzugsschichten auf der pharmazeutischen Darreichungsform aufgebracht werden, wobei eine hiervon eine pH-unabhängige, wasserlösliche Überzugsschicht darstellt (Subcoating) und die zweite eine (konventionelle) magensaftresistente Überzugsschicht darstellt (Topcoating).

Es hat sich überraschend herausgestellt, dass durch den erfindungsgemäßen Ansatz auf phthalathalige Komponenten vollständig verzichtet werden kann, wobei die Zerfallsergebnisse der pharmazeutischen Zusammensetzung mit den konventionellen Zusammensetzungen vergleichbar oder sogar deutlich verbessert sind.

Die vorliegende pharmazeutische Zusammensetzung erfüllt darüber hinaus alle Laufzeitspezifikationen, OOS-Ergebnisse bezüglich des Zerfalls treten nicht in nennenswertem Umfang auf. Damit gelingt es mit der vorliegenden pharmazeutischen Zusammensetzung, eine untoxische pharmazeutische Zusammensetzung zur peroralen Verabreichung bereitzustellen, die den gängigen Spezifikationen der Arzneibücher entspricht.

### Ausführliche Beschreibung der Erfindung.

Gemäß eines ersten Aspekts betrifft die vorliegende Erfindung eine pharmazeutische Zusammensetzung zur peroralen Verabreichung, die ein Erdalkalimetall- oder Alkalimetallcarbonat eingebettet in einer lipophilen Matrix aufweist, wobei die pharmazeutische Zusammensetzung wenigstens eine pH-unabhängige, wasserlösliche Überzugsschicht und eine darauf aufgebrachte magensaftresistente Überzugsschicht aufweist.

Mit anderen Worten umfasst die pharmazeutische Zusammensetzung der vorliegenden Erfindung wenigstens zwei Überzugsschichten, kann jedoch abhängig vom beabsichtigten Zweck noch ein oder mehrere zusätzliche Überzugsschichten aufweisen.

Das Freisetzungsverhalten der vorliegenden pharmazeutischen Zusammensetzung entspricht und wird durchgeführt unter den im Europäischen Arzneibuch (Ph. Eur.) angegebenen Vorschriften, siehe insbesondere 2.9.1 bis 2.9.3, sowie die einschlägige Kommentierung hierzu.

Als Erdalkalimetall- oder Alkalimetallcarbonate kommen vorwiegend insbesondere Natriumcarbonat, Magnesiumcarbonat, Calciumcarbonat, Magnesiumhydrogencarbonat, Calciumhydrogencarbonat und Natriumhydrogencarbonat, sowie Gemische hiervon in Betracht. Natriumhydrogencarbonat wird besonders bevorzugt. Die Einzeldosen umfassen hier insbesondere 300 mg bis 900 mg, besonders bevorzugt 500 mg und 840 mg. Es sei darauf hingewiesen, dass der Begriff Erdalkalimetall- bzw. Alkalimetallcarbonate gemäß der oben angesprochenen Definition nicht nur Carbonate selbst, sondern gerade auch Hydrogencarbonate umfasst.

In der erfindungsgemäßen pharmazeutischen Zusammensetzung liegt ein Erdalkalimetall- oder Alkalimetallcarbonat in einer lipophilen Matrix eingebettet vor. Solche lipophilen Matrix-Materialien bestehen typischerweise aus klassischen hydrophoben Substanzen, wie beispielsweise Triglyceriden (beispielsweise Vitokan^{®}) oder auch ähnlichen Substanzen, die eine ausreichende Diffusionsbarriere im Gastrointestinaltrakt bereitstellen. Allgemein sind beispielsweise Matrixmaterialen aus (halbsynthetischen) Triglyceriden, Diglyceriden, Glycerinmonostearat, Paraffinen, natürlichen / (teil-)hydrierten Fetten / Ölen und/oder Wachsen anzusprechen. Der Fachmann ist auf Grundlage der speziellen Anforderungen des Einzelfalles und auf Grund seines Fachwissens dazu in der Lage, die jeweils geeigneten lipophilen Matrixsubstanzen zu bestimmen und dem Zweck entsprechend einzusetzen. Beispielsweise wird sich bei der Herstellung von lipophilen Matrix-Zusammensetzungen zum Einsatz in Weichgelatinekapseln ein Gemisch aus Rapsöl, Lecithin, (gelbem) Bienenwachs, hydriertem Sojaöl, sowie teilweise hydriertem Sojaöl anbieten. Das Gewichtsverhältnis des lipophilen Matrixbestandteils zu den eingesetzten Erdalkalimetall- oder Alkalimetallcarbonaten kann abhängig von den Gegebenheiten des Einzelfalls schwanken, jedoch ist ein Gewichtsverhältnis von 30 zu 70 bis 70 zu 30 jedoch als durchaus realistisch einzustufen.

Bezüglich weiterer Informationen zu Matrix-Materialen, die in dem Kontext der vorliegenden Erfindung zur Herstellung verwendet werden können, wird auf die einschlägige Fachliteratur verwiesen, insbesondere Bauer/Frömming/Führer "Lehrbuch der pharmazeutischen Technologie" (wissenschaftliche Verlagsgesellschaft; Auflage 8, März 2006).

Die lipophile Matrix, in die die Erdalkalimetall- oder Alkalimetallcarbonate eingebettet sind, wird vorzugsweise in Weich- oder Hartgelatinekapseln abgefüllt. Diese weisen aufgrund ihres Aufbaus Unebenheiten auf (beispielsweise Nähte), die sich beim Überziehen als Schwachstellen erweisen können und so zu nicht spezifikationskonformer Freisetzung führen können. Daneben kommen auch andere Darreichungsformen in Betracht, die beispielsweise aufgrund ihrer speziellen dreidimensionalen Form schwer zu überziehen sind (z.B. scharfkantige Tabletten).

Danach wird zunächst eine pH-unabhängige, wasserlösliche Überzugsschicht auf die Hart- oder Weichgelatinekapsel aufgebracht. Diese Überzugsschicht besteht vorzugsweise aus einem Gemisch aus Hypromellose und Hydroxypropylcellulose, es können jedoch auch andere Überzugsmaterialien eingesetzt werden, die sich nach kurzer Quellzeit in wässriger Umgebung rasch und pH-unabhängig auflösen. Das Freisetzungsprofil der pharmazeutischen Zusammensetzung selbst wird durch diese erste Überzugsschicht nicht oder nur kaum beeinflusst. Weitere wasserlösliche Überzugsmaterialien, die zur Verwendung in der vorliegenden Erfindung geeignet sind, sind Methylcellulose, Hydroxyethycellulose, NatriumCarboxymethylcellulose, insofern die Wasserlöslichkeit abhängig vom durchschnittlichen Substitutionsgrad gewährleistet ist.

Bezüglich der Auswahl weiterer Überzugsmaterialien wird auf die oben angegebene Fachliteratur verwiesen (Bauer/Frömming/Führer "Lehrbuch der pharmazeutischen Technologie" (wissenschaftliche Verlagsgesellschaft; Auflage 8, März 2006); und Rudolf Voigt "Pharmazeutische Technologie" (11. Auflage 2010).

Wie üblich wird ein derartiges Überzugsmaterial als wässrige Suspension auf den Arzneimittelkern aufgebracht, beispielsweise eine Suspension folgender Zusammensetzung:
Hydroxypropylmethylcellulose (Hypromellose) 12,50 Gewichtsteile, Hydroxypropylcellulose 10,00 Gewichtsteile, Talkum 2,00 Gewichtsteile, Polyethylenglycol 0,50 Gewichtsteile, sowie gereinigtes Wasser. Gereinigtes Wasser dient hier lediglich als Suspensionsgrundlage und wird nach dem Trocknen der Überzugsschicht entzogen.

Nach dem vollständigen Trocknen der ersten pH-unabhängigen, wasserlöslichen Überzugsschicht wird sodann eine phthalat-freie magensaftresistente Überzugsschicht aufgebracht. Hierbei kommen insbesondere magensaftresistente Überzugsmaterialien in Betracht, die aus Hydroxypropylmethylcellulosesuccinat, Carboxymethylethylcellulose und/oder Methacrylsäure-Methylmethacrylat-Copolymer [1:1] ausgewählt sind. Insbesondere das Überzugsmaterial Eudragit L30 D-55 kann hier eingesetzt werden.

Eine besonders bevorzugte Rezeptur zur Herstellung des magensaftresistenten Lacks für die zweite Überzugsschicht setzt sich aus Folgendem zusammen:
Eudragit L30 D-55 40,00 Gewichtsteile, Polysorbat 80 3,41 Gewichtsteile, Natriumlaurylsulfat 1,04 Gewichtsteile, Propylenglycol 8,00 Gewichtsteile, Glycerolmonostearat 4,00 Gewichtsteile, Polysorbat 80 0,33 Gewichtsteile, sowie gereinigtes Wasser.

Insbesondere weisen die beiden Überzugsschichten der pharmazeutischen Zusammensetzung in qualitativer Hinsicht die oben angegebenen Inhaltsstoffe auf (können von den angegebenen Gewichtsteilen jedoch abweichen).

In einer bevorzugten Ausführungsform liegt das Gewichtsverhältnis von magensaftresistentem zu wasserlöslichem Überzug in einem Bereich von zwischen 3:1 bis 2:1 vor.

Gemäß eines zweiten Aspektes findet die vorliegende pharmazeutische Zusammensetzung Verwendung bei der Behandlung einer metabolischen Azidose und/oder einer Übersäuerung des Blutes bei chronischer Niereninsuffizienz.

Gemäß eines weiteren Aspektes betrifft die vorliegende Erfindung ein Verfahren zur Herstellung einer pharmazeutischen Zusammensetzung wie oben definiert, das die folgenden Schritte umfasst:
(a) Einbringen und homogenes Verteilen von Erdalkalimetall- oder Alkalimetallcarbonaten in einer lipophilen Matrix;
(b) Einbringen des Gemisches in eine Hart- oder Weichgelatinekapsel;
(c) Aufbringen einer pH-unabhängigen, wasserlöslichen Überzugsschicht und
(d) darauf Aufbringen einer magensaftresistenten Überzugsschicht.

Die Komponenten sind vorzugsweise wie oben definiert.

### Kurze Beschreibung der Figuren

Figur 1 beschreibt das *in vitro* Freisetzungsverhalten einer Zusammensetzung des Stands der Technik ermittelt durch sequentielle Freisetzung in der Paddle-Apparatur gemäß Europäischem Arzneibuch.
Figuren 2-4 beschreiben erfindungsgemäße Beispiele mit einem dualen Überzug aus einer wasserlöslichen und einer magensaftresistenten Schicht auf dem Arzneimittelkern.
Figur 5 zeigt die *in vitro* Freisetzung einer pharmazeutischen Zusammensetzung des Stands der Technik.
Figuren 6 und 7 beschreiben die *in vitro* Freisetzung von zwei Ausführungsformen gemäß der vorliegenden Erfindung.

Alle vorgenannten Freisetzungsversuche basieren auf einer Arzneiform mit 500 mg Natriumhydrogencarbonat pro Arzneiform.

Figuren 8 und 9 beschreiben einen Freisetzungsversuch einer pharmazeutischen Zusammensetzung gemäß des Stands der Technik mit 840 mg Natriumhydrogencarbonat pro Einheit.

Die Figuren 10-13 beschreiben Freisetzungsexperimente von pharmazeutischen Zusammensetzungen gemäß der vorliegenden Erfindung mit jeweils 840 mg Natriumhydro gencarbonat.

### Beispiele

### I. Herstellung einer erfindungsgemäßen pharmazeutischen Zusammensetzung:

Eine Zusammensetzung gemäß der vorliegenden Erfindung wurde entsprechend der folgenden Spezifikationen hergestellt:

| **Inhaltsstoffe** | **Menge in mg** | **Standards** |
|---|---|---|
| **A. Wirkstoff** | | |
| Natriumhydrogencarbonat | 500,00 | Ph. Eur. |
| **B. weitere Inhaltsstoffe** | | |
| *Kapselfüllung* | | |
| raffiniertes Rapsöl | 280,00 | Ph. Eur. |
| Lecithin | 10,00 | NF, RPS-monogr. 5082 |
| gelbes Bienenwachs | 15,00 | Ph. Eur. |
| Sojaöl, hydriert | 15,00 | Ph. Eur. |
| teilweise hydriertes Sojaöl | 60,00 | DAB, RPS-monogr. 5275 |
| **Füllgewicht** | **880,00** | |
| *Kapselhülle:* | | |
| Trockensubstanz Anidrisorb | 94,24 - 110,62 | RPS-monogr. 7020 |
| 85/70: | (102,43) | |
| bestehend aus | | |
| Sorbitol | 27,72 - 52,06 | |
| Sorbitanhydriden | 22,17 - 39,04 | |
| Mannitol | 0,00 - 7,81 | |
| Höheren Polyolen | 13,86 - 24,73 | |
| Gelatine (als wasserfreie | 164,15 - 192,70 | Ph. Eur. |
| Substanz) | (178,42) | |
| Rotes Eisenoxid, E 172 | 1,12 - 1,31 (1,22) | RPS-monogr. 8031 |
| Glycerol (als wasserfreie | 2,85 - 3,35 | Ph. Eur. |
| Substanz aus Glycerol 85%) | (3,10) | |
| Titandioxid E171 | 2,24 - 2,62 (2,43) | Ph. Eur., RPS-monogr. 4000 |
| Salzsäure (theor. berechnet als | 0,38 - 0,45 | Ph. Helv. |
| wasserfreie Substanz, aus HCl 25%) | (0,42) | |
| Gereinigtes Wasser | 30,62 - 35,95 33,29 | Ph. Eur. |
| **Gewicht Hülle:** | **295,60 - 347,00 (321,29)** | |

| | | |
|---|---|---|
| **wasserlösl. Überzugsschicht** | | |
| Hypromellose | | Ph. Eur., USP |
| Hydroxypropylcellulose | 12,50 | Ph. Eur., US NF |
| Talkum | 10,00 | Ph. Eur., USP |
| Polyethylenglycol | 2,00 | Ph. Eur., US NF |
| gereinigtes Wasser | 0,50 | |
| **Gewicht wasserlösl. Besch.** | **25,00** | |
| **magensaftresistente Überzugsschicht:** | | |

| | | |
|---|---|---|
| Methacrylsäure- | 40,00 | Ph. Eur. |
| Ethylacrylatcopolimer (1:1) | | |
| Polysorbat 80 | 3,41 | Ph. Eur. |
| Natriumlaurylsulfat | 1,04 | Ph. Eur. |
| Propylenglycol | 8,00 | Ph. Eur. |
| Glycerolmonostearat | 4,00 | Ph. Eur. |
| Polysorbat 80 | 0,33 | Ph. Eur. |
| gereinigtes Wasser | | |
| **Gewicht dieser Schicht** | **56,78** | |
| **Gesamtes Kapselgewicht** | | |
| | **1257,4 - 1308,8** | |

### II. Herstellung der pharmazeutischen Zusammensetzung gemäß Stand der Technik

Die Herstellung erfolgte wie in I., außer dass eine andere Zusammensetzung für die Überzugsschicht verwendet wurde.

| **Inhaltsstoffe** | **Menge [mg]** | **Standards** |
|---|---|---|
| *Magensaftresistente Überzugsschicht* | | |
| Glycerol | 1,22 - 1,50 | Ph. Eur. |
| (als wassefreie Substanz aus Glycerol 85%) | | |
| Citronensäure, wasserfrei | 2,88 - 3,52 | Ph. Eur. |
| Hypromellose (Visc. 40-60 mPa) | 0,72 - 0,88 | Ph. Eur. |
| Hypromellose (Visc. 13-18 mPa) | 5,04 - 6,16 | Ph. Eur. |
| Hypromellose (Visc. 4-6 mPa) | 8,64 - 10,56 | Ph. Eur. |
| Dibutylphthalat | 8,10 - 9,90 | Ph. Eur. |
| Hypromellosephthalat | 40,50 - 49,50 | Ph. Eur. |
| Gereinigtes Wasser | 0,22 - 0,26 | Ph. Eur. |

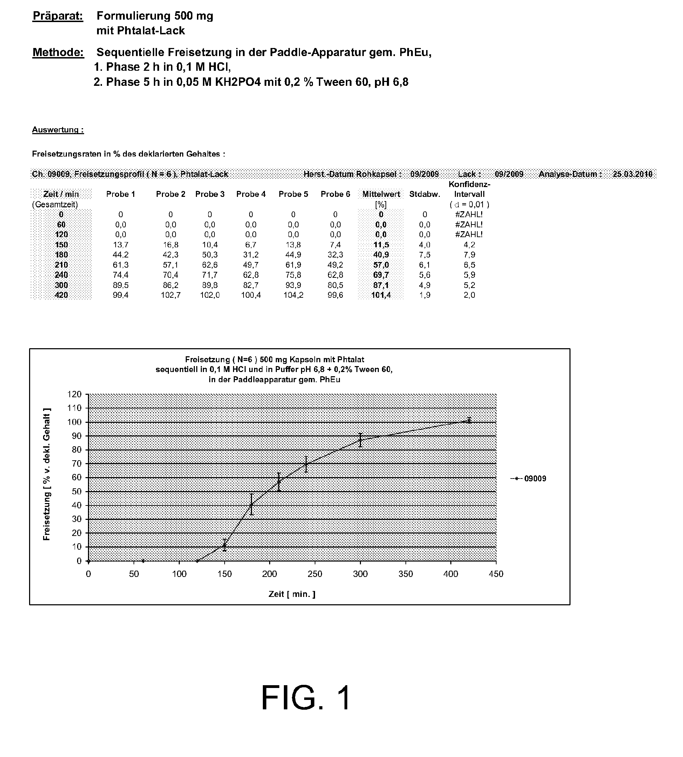

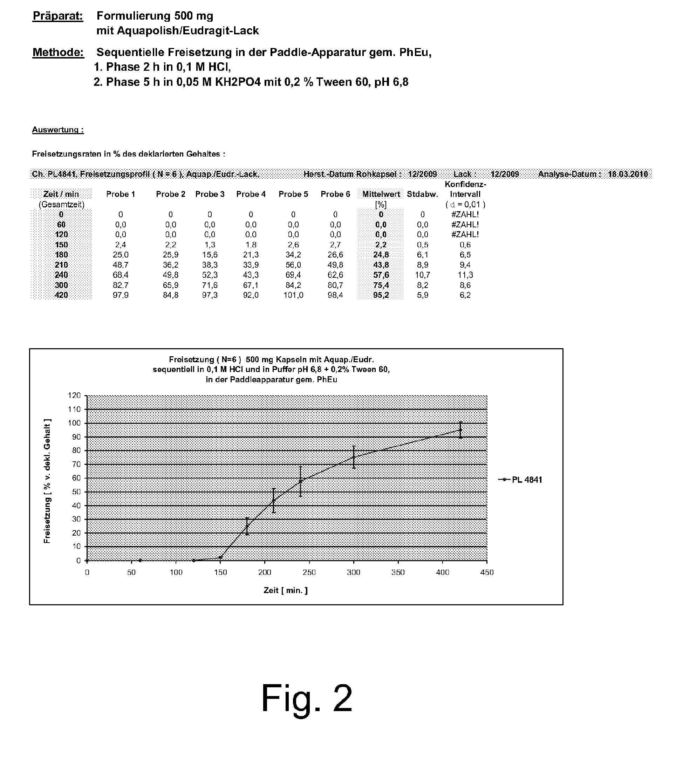

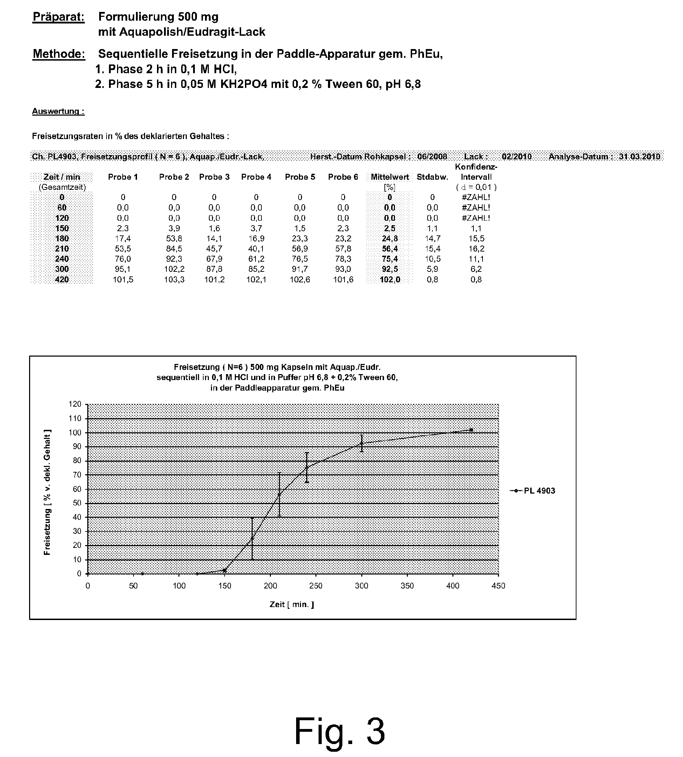

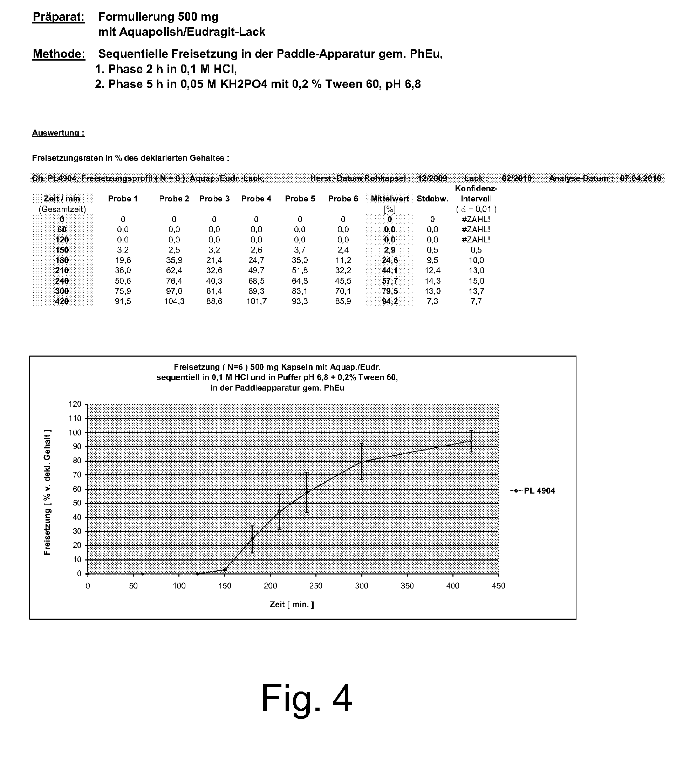

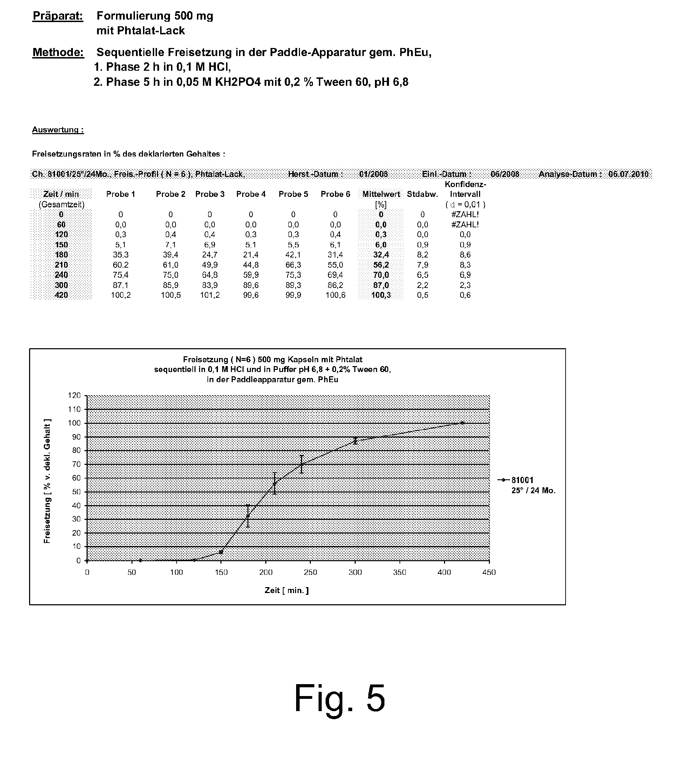

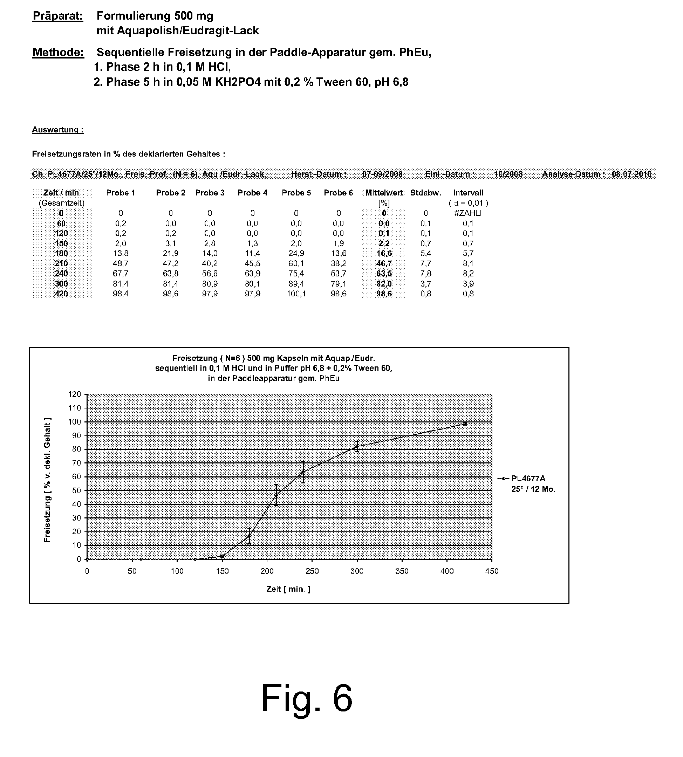

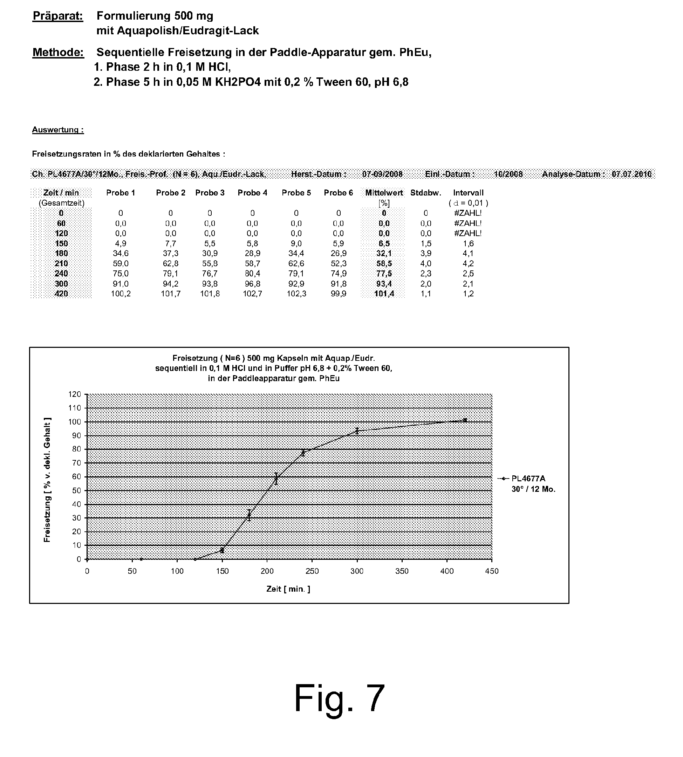

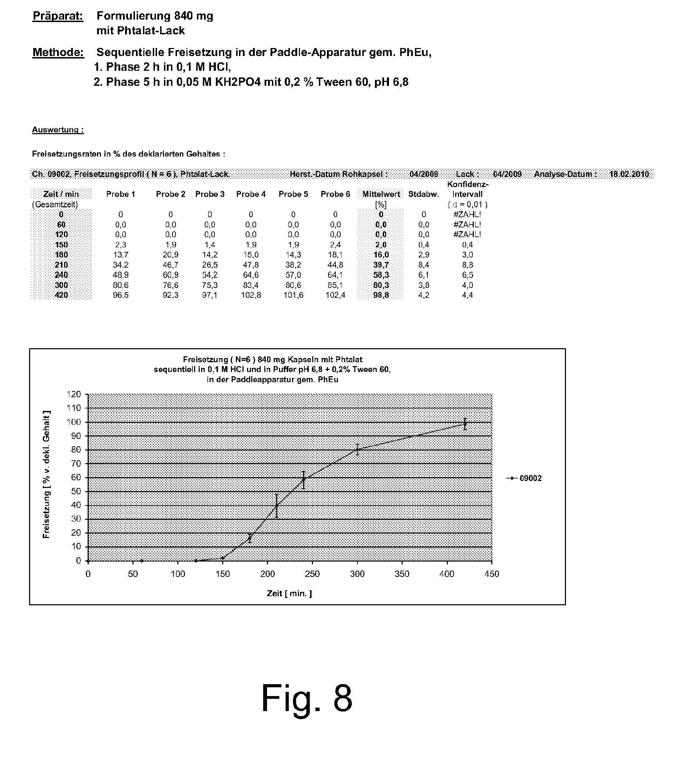

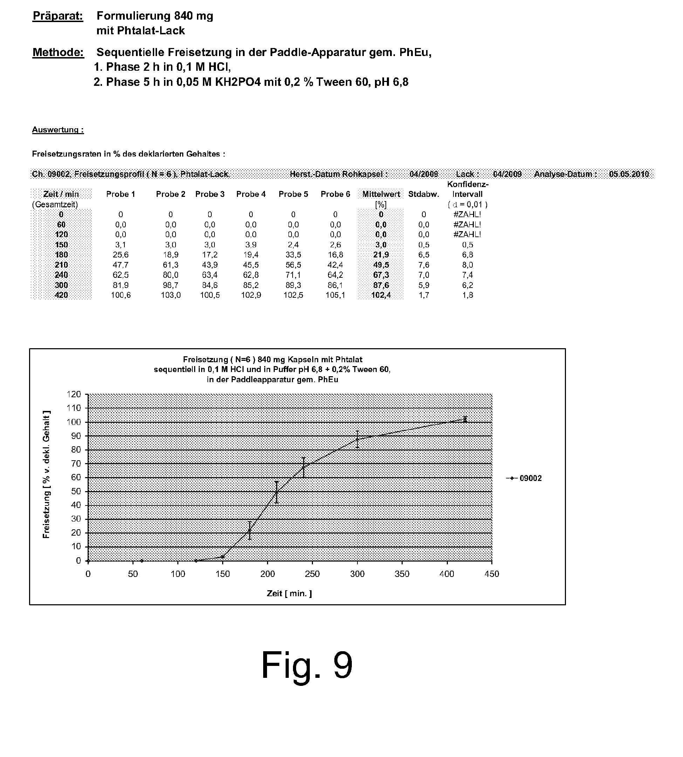

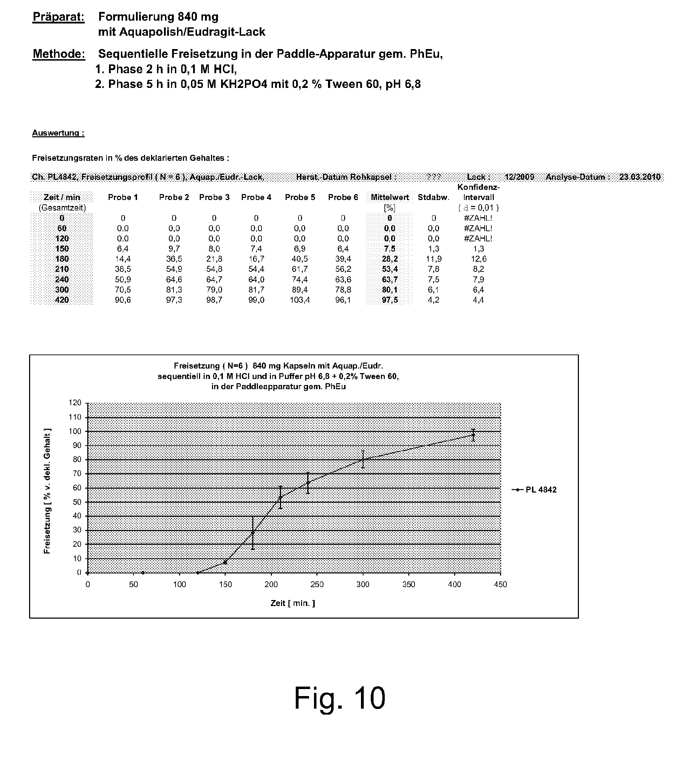

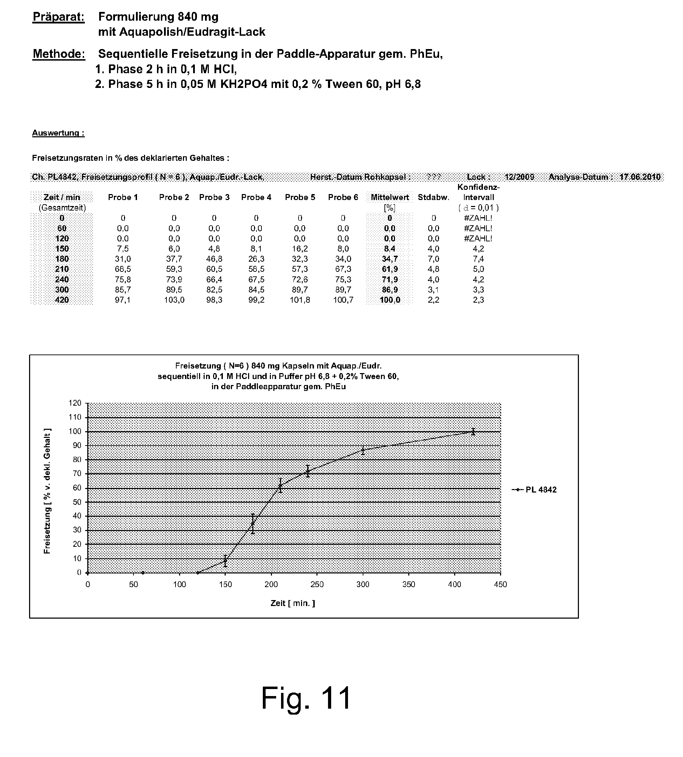

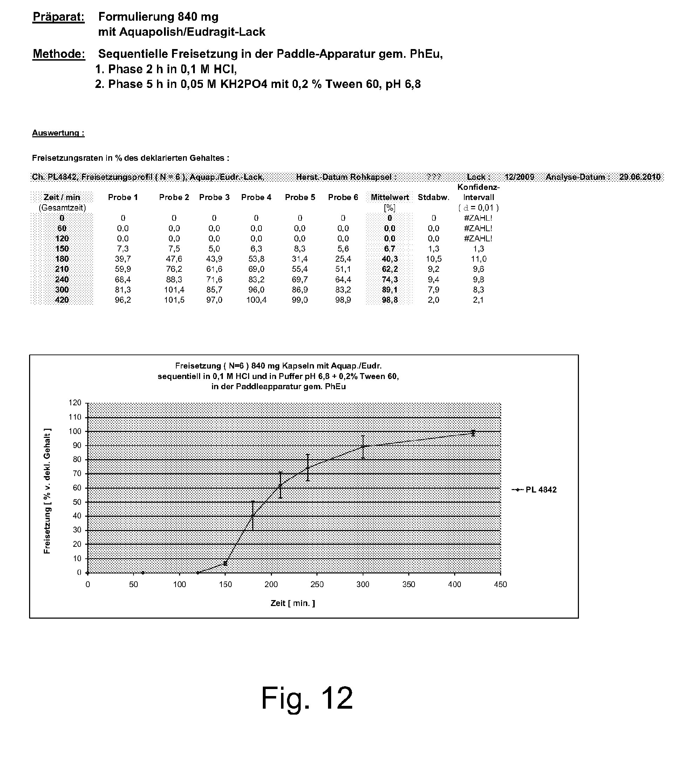

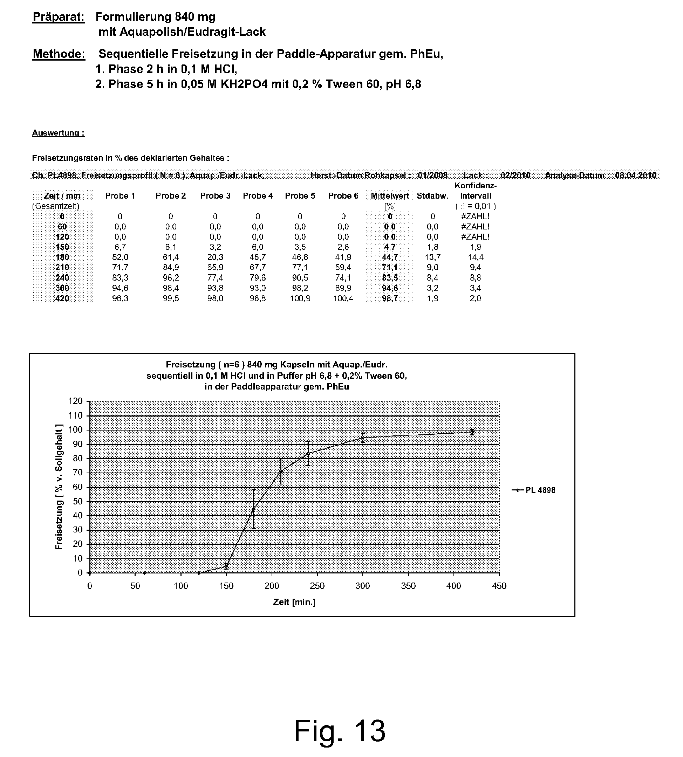

Wie den Figuren 1-13 zu entnehmen ist, weist die erfindungsgemäße Zusammensetzung einen vollkommen phthalatfreien Überzug auf, sodass in Hinblick auf die Toxizität alle Probleme ausgeräumt sind.

Daneben sind die Zerfallsergebnisse der erfindungsgemäßen Zusammensetzungen im Vergleich zum Stand der Technik deutlich verbessert oder annähernd vergleichbar und weisen deutlich reduzierte Filmundichtigkeiten im Vergleich zum phthalatfreien Monocoating auf.

Damit können mit der Zusammensetzung gemäß der vorliegenden Erfindung untoxische, phthalatfreie Überzugsmaterialien bereitgestellt werden, die insbesondere in Zusammenhang von anti-azidotischen Arzneimitteln eine sichere magensaftresistente Beschichtung aufweisen, die die Freisetzung des alkalischen Wirkstoffes im Magen mit Sicherheit vermeiden können.

## Patentansprüche

1. Pharmazeutische Zusammensetzung zur peroralen Verabreichung, die ein Erdalkalimetall- oder Alkalimetallcarbonat eingebettet in einer lipophilen Matrix aufweist,
**dadurch gekennzeichnet,**
**dass** die pharmazeutische Zusammensetzung wenigstens
a) eine pH-unabhängige, wasserlösliche Überzugsschicht, und
b) eine darauf aufgebrachte magensaftresistente Überzugsschicht aufweist.

2. Pharmazeutische Zusammensetzung nach Anspruch 1, wobei das Erdalkalimetall- oder Alkalimetallcarbonat aus Natriumcarbonat, Magesiumcarbonat, Magesiumhydrogencarbonat, Calciumhydrogencarbonat, Natriumhydrogencarbonat und Calciumcarbonat, sowie Mischungen hiervon ausgewählt ist.

3. Pharmazeutische Zusammensetzung nach Anspruch 2, wobei das Alkalimetallcarbonat Natriumhydrogencarbonat ist.

4. Pharmazeutische Zusammensetzung nach Anspruch 1 - 3, wobei die Zusammensetzung die lipophile Matrix in einer Hart- oder Weichgelatinekapsel enthält.

5. Pharmazeutische Zusammensetzung nach einem oder mehreren der vorhergehenden Ansprüche, wobei die pharmazeutische Zusammensetzung eine Menge von 200 bis 1.500 mg Natriumhydrogencarbonat aufweist.

6. Pharmazeutische Zusammensetzung nach Anspruch 5, die von 300 bis 900 mg Natriumhydrogencarbonat aufweist.

7. Pharmazeutische Zusammensetzung nach einem oder mehreren der vorhergehenden Ansprüche, wobei der magensaftresistente Überzug aus Hydroxypropylmethylcellulosesuccinat, Carboxymethylethylcellulose, und/oder Methacrylsäure-Methylmethacrylat-Copolymer [1:1] ausgewählt ist.

8. Pharmazeutische Zusammensetzung nach einem oder mehreren der vorhergehenden Ansprüche, wobei das lipophile Matrixmaterial aus (halbsynthetischen) Triglyceriden, Diglyceriden, Glycerinmonostearat, Paraffinen, natürlichen / (teil-)hydrierten Fetten / Ölen und/oder Wachsen ausgewählt ist.

9. Pharmazeutische Zusammensetzung nach einem oder mehreren der vorhergehenden Ansprüche, wobei der magensaftresistente Überzug Methacrylsäure-Ethylacrylat-Copolymer (1:1) umfasst und die wasserlösliche Schicht ein Gemisch aus Hypromellose und Hydroxypropylcellulose aufweist.

10. Pharmazeutische Zusammensetzung nach einem oder mehreren der vorhergehenden Ansprüche, wobei die wasserlösliche Schicht Folgendes umfasst:
Hypromellose
Hydroxypropylcellulose,
Talkum,
Polyethyleneglycol,
gereinigtes Wasser
und wobei die magensaftresistente Schicht Folgendes aufweist:
Methacrylsäure-Ethylacrylat-Copolymer (1:1),
Polysorbat 80,
Natriumlaurylsulfat,
Propylenglycol,
Glycerolmonostearat,
gereinigtes Wasser.

11. Pharmazeutische Zusammensetzung nach einem oder mehreren der vorhergehenden Ansprüche, wobei das Gewichtsverhältnis von magensaftresistentem zu wasserlöslichem Überzug zwischen 3:1 bis 2:1 beträgt.

12. Pharmazeutische Zusammensetzung nach einem oder mehreren der vorhergehenden Ansprüche, wobei die Überzugsschichten phthalatfrei sind.

13. Verwendung einer pharmazeutischen Zusammensetzung nach einem oder mehreren der vorhergehenden Ansprüche zur Behandlung einer metabolischen Acidose und/oder einer Übersäuerung des Blutes bei chronischer Niereninsuffizienz.

14. Verfahren zur Herstellung einer pharmazeutischen Zusammensetzung nach einem oder mehreren der vorhergehenden Ansprüche, das die folgenden Schritte umfasst:
a) Einbringen und homogenes Verteilen von Erdalkalimetall- oder Alkalimetallcarbonat in einer lipophilen Matrix;
b) Einbringen des Gemisches in eine Hart- oder Weichgelatinekapsel;
c) Aufbringen einer pH-unabhängigen, wasserlöslichen Überzugsschicht; und
d) darauf Aufbringen einer magensaftresistenten Überzugsschicht.
